# EUROPÄISCHE PATENTSCHRIFT

(11) **EP 1 905 460 B1**
(45) Veröffentlichungstag und Bekanntmachung des Hinweises auf die Patenterteilung: **29.09.2010**
(21) Anmeldenummer: 07013749.2
(22) Anmeldetag: 13.07.2007
(51) Int. Cl.: A61L 27/02, A61L 27/50

(54) **Knochenersatzmaterial**
Bone replacement material
Matériau de remplacement osseux

(30) Priorität: 09.08.2006 DE 102006037362
(43) Veröffentlichungstag der Anmeldung: 02.04.2008
(73) Patentinhaber: Heraeus Kulzer GmbH, 63450 Hanau (DE)
(72) Erfinder: Kühn, Klaus-Dieter, Dr., 35041 Marburg (DE); Vogt, Sebastian, Dr., 99092 Erfurt (DE)
(74) Vertreter: Kühn, Hans-Christian

(56) Entgegenhaltungen:
- WO-A-03/028779
- US-A- 5 618 549
- US-A1- 2006 013 857

## Beschreibung

Gegenstand der Erfindung ist ein Knochenersatzmaterial. Dieses Knochenersatzmaterial soll insbesondere zur Augmentation von humaner Spongiosa geeignet sein.

Für die Auffüllung von Knochendefekten ist nach wie vor die autologe Spongiosa der so genannte Golds-Standard (J. Jerosch, A. Bader, G. Uhr: Knochen curasan Taschenatlas spezial. Thieme Verlag 2002). Mit autologer Spongiosa werden gegenwärtig die besten Ergebnisse erreicht. Die Verwendung von autologer Spongiosa ist naturgemäß hinsichtlich ihres maximal gewinnbaren Volumens limitiert. Es liegt daher nahe, die autologe Spongiosa mit geeigneten Knochenersatzwerkstoffen zu augmentieren, um größere Knochendefekte auffüllen zu können. Üblich ist die Vermischung von 2 Volumenteilen Spongiosa mit einem Volumenteil Knochenersatzwerkstoff.

Es sind eine Vielzahl von granulären Keramiken aus Calciumphosphaten, wie Hydroxylapatit, β-Tricalciumphosphat und α-Tricalciumphosphat, im Medizinproduktemarkt bekannt (J. M. Rueger, W. Linhart, D. Sommerfeldt: Biologische Reaktionen auf Kalziumphosphatkeramik-Implantationen. Tierexperimentelle Ergebnisse. Orthopäde 27 (1998) 89-95.). Beispielhaft sind dafür die in Deutschland häufig benutzten Knochenersatzmaterialien Endobone®, Catcibone®, Biobase®, BETABASE® und Cerasorb®. Eine interessante Alternative bilden pastenförmige Knochenersatzmaterialien, die auf nanopartikulären Hydroxylapatit beruhen und unter dem Namen Ostim® im Markt bekannt sind (EP0664133 A1). Nanopartikuläres Hydroxylapatit wird dabei in Wasser so suspendiert, dass spritzbare Pasten entstehen. Voraussetzung für dieses Material ist jedoch die Verfügbarkeit von nanopartikulärem Hydroxylapatit, der in einem speziellen Herstellungsprozess synthetisiert werden muss. Durch diesen Herstellungsprozess sind naturgemäß die Fertigungskosten gegenüber einem Material höher, das in pharmazeutischer Qualität in Bulk-Quantitäten kommerziell verfügbar ist. Daneben sind auch Pasten bekannt, die eine Kombination von nanopartikulärem Hydroxylapatit und Calciumsulfat darstellen

(EP1301219 A1). Auch hierbei wird nanopartikuläres Hydroxylapatit eingesetzt, das jedoch nur kostenintensiv herstellbar ist.

US20030055512A1 betrifft ebenfalls eine injizierbare Paste enthaltend bioabbaubare Calciumverbindungen wie Calciumsulfat, Hydroxyapatit und Tricalciumphosphat. Die Härtungszeit im wässrigen System ist einstellbar.

Laut EP1243257A1 wird ein amphiphiles organisches Sulfat, Sulfamat, Sulfonat, Disulfat, Disulfonat oder Trisulfonat einer Aminoglycosid, Lincosamid, 4-Chinolon oder Tetracyclin Antibiotikum-Zubereitung einverleibt, um eine verzögerte Wirkstofffreisetzung zu erzielen. W003028779A1 offenbart ein Knochenersatzmaterial, welches durch Nadeln injizierbar ist und partikuläres Calciumcarbonat und ein Hämostyptikum (Natrium Alginat) in einer wässrigen, isotonischen Lösung (PBS) enthält (D1: S. 4 Z. 4-8, S. 4, Z. 26 - S. 5, Z. 15 und S. 5, Z. 25 - S.6, Z. 12).

US-A-5 618 549 offenbart ein Knochenersatzmaterial, das pastös und durch Nadeln injizierbar ist. Das Material enthält partikuläres Calciumcarbonat (40-97 %) und ein Hämostyptikum (Kollagen) in einer wässrigen, isotonischen (PBS) Lösung (D2: Sp. 2, Z. 32-51 und Sp. 3, Z. 29-62).

Der Erfindung liegt die Aufgabe zu Grunde, ein kostengünstiges, einfach zu fertigendes Knochenersatzmaterial bereitzustellen, das bequem in Knochenkavitäten unterschiedlichster Gestalt und Größe eingebracht werden kann.

Die Aufgabe wird erfindungsgemäß durch die Merkmale des Anspruchs 1 gelöst. Vorzugsweise Ausgestaltungen sind in den Unteransprüchen angegeben. Die Aufgabe wird dadurch gelöst, dass das Knochenersatzmaterial Calciumcarbonat enthält, welches als partikuläres Calciumcarbonat in einer wässrigen Lösung, die mindestens ein in Wasser lösliches Hämostyptikum enthält, suspendiert ist, wobei die wässrige Lösung eine solche Menge Hämostyptikum enthält, daß die wässrige Lösung isotonisch ist. Das humane Blut hat einen osmotischen Druck bei 37 °C von 7,5 bar. Isotonisch zum Blut ist die wässrige Lösung bei einer Osmolalität von 288 mOsmol. Im folgenden wird unter dem Begriff isotonisch verstanden, dass die Osmolalität der wässrigen Lösung bei 37°C im Bereich von 250-300 mOsmol ist.

Nach erfolgter Implantation zeigt das Knochenersatzmaterial einerseits eine Volumenstabilität über einen Zeitraum von mehreren Tagen bis Wochen und andererseits erlangt das Knochenersatzmaterial innerhalb kurzer Zeit eine solche Formstabilität, dass es innerhalb der ersten Tage nach der Implantation nicht durch Fließen migrieren kann. Das Knochenersatzmaterial verursacht im Knochendefekt während der Resorption keine Säuerung (pH-Absenkung). Weiterhin sind die Bestandteile des Knochenersatzmaterials auch natürliche Bestandteile des humanen Organismus, so dass Unverträglichkeiten und toxische Effekte vermieden werden.

Calciumcarbonat ist in Kohlendioxid-freiem Wasser schwer löslich und zeigt eine ausgeprägte Pufferwirkung gegenüber Säuren. Es ist nicht cytotoxisch und es sind auch keine systemisch toxischen Effekte bekannt. Calciumcarbonat ist aus Calcium-lonen und Carbonat-lonen aufgebaut. Calcium-lonen und Carbonat-Ionen sind natürliche Bestandteile des humanen Organismus. Calcium-lonen kommen unter anderem in der anorganischen Substanz des Knochens, im Carbonatapatit, und in der Hartsubstanz der Zähne vor. Carbonat-lonen sind ein Bestandteil des Kohlendioxid-Hydrogenarbonat-Carbonat-Puffers im Blut. Dieser Puffer gewährleistet die Konstanz des pH-Wertes im humanen Blut. Carbonat-Ionen stehen in wässriger Lösung unter Mitwirkung von Protonen im chemischen Gleichgewicht mit gasförmigen Kohlendioxid. Carbonat-lonen werden in Form von Kohlendioxid über die Lunge aus dem humanen Organismus ausgeschieden. In Kohlendioxid-freiem Wasser ist Calciumcarbonat schwer löslich. So lösen sich in Kohlendioxid-freiem Wasser bei 20 °C nur 14 mg Calciumcarbonat pro Milliliter Wasser (Römpp-Lexikon Chemie. Hrsg.: J. Falbe; M. Regitz, 10. völlig überarbeitete Auflage, Band 1, Thieme Verlag 1996, S. 574). Jedoch in Gegenwart von im Wasser gelösten Kohlendioxid löst sich Calciumcarbonat unter Freisetzung von Calcium-Ionen und Hydrogencarbonat-Ionen. Bei 20 °C werden in Kohlendioxid-gesättigtem Wasser 0,85 g Calciumcarbonat pro Milliliter Wasser gelöst (Römpp-Lexikon Chemie. Hrsg.: J. Falbe; M. Regitz, 10. völlig überarbeitete Auflage, Band 1, Thieme Verlag 1996, S. 574). Trotz seiner Schwerlöslichkeit in Wasser ist dadurch eine Auflösung des Calciumcarbonats im humanen Organismus möglich, weil im Hart- als auch im Weichgewebe immer Kohlendioxid in gelöster Form vorhanden ist.

Calciumcarbonat hat gegenüber nanopartikulärem Hydroxylapatit den wirtschaftlichen Vorteil, dass ist in technisch relevanten Mengen in pharmazeutischer Qualität kostengünstig verfügbar ist.

Das Knochenersatzmaterial ist vorzugsweise pastös. Die Erfindung basiert einerseits darauf, dass partikuläres Calciumcarbonat mit Wasser, bei einem Calciumcarbonat-Gehalt von vorzugsweise 55 bis 67 Masseprozent, pastenförmige Gemische bildet, die problemlos spritzbar sind, und andererseits darauf, dass durch ein in dem Wasser der Paste gelöstes Hämostyptikum, die Blutgerinnung nach Kontakt des pastenförmigen Knochenersatzmaterials mit Blut initiiert wird. Dadurch ist es möglich, dass das insbesondere pastenförmige Knochenersatzmaterial durch das sich dabei bildende vernetzte Fibrin räumlich fixiert wird und infolgedessen Migrationsprozesse des Knochenersatzmaterials für einen Zeitraum von mehreren Tagen verhindert beziehungsweise verzögert werden. Wesentlich ist dabei, dass das Hämostyptikum eine isotonische Lösung mit dem Wasser bildet. Eine Paste mit einem Calciumcarbonat-Gehalt von 60 Masseprozent erwies sich als optimal hinsichtlich der Spritzbarkeit.

Vom Calciumcarbonat sind die Modifikationen Vaterit, Calcit und Aragonit bekannt, die durch Einstellung entsprechender Syntheseparameter, wie zum Beispiel der Reaktionstemperatur, synthetisiert werden können. Vorteilhaft ist, dass die bekannten Modifikationen des Calciumcarbonats oder Gemische dieser Modifikationen im erfindungsgemäßen Knochenersatzmaterial enthalten sein können.

Weiterhin vorteilhaft ist, dass das Calciumcarbonat eine Partikelgröße bevorzugt von 1-50 µm hat. Ebenfalls möglich ist die Verwendung von kleineren Calciumcarbonat-Partikeln. Diese können auch eine Größe im Nanometerbereich haben.

Ein oder mehrere Stoffe aus der Gruppe Calciumchlorid, Calciumacetat, Calciumlactat, werden erfindungsgemäß als Hämostyptikum eingesetzt. Es ist eine allgemein bekannte Tatsache, dass gelöste Calcium-lonen die Blutgerinnung beschleunigen können. Calcium-lonen sind ein essentieller Bestandteil an mehreren Stellen der Gerinnungskaskade. Sie wirken mit bei der Aktivierung von Faktor VII und Faktor IX und damit bei der Bildung des Prothrombinaktivators. Calcium-lonen sind weiterhin essentiell bei der Einwirkung von Thrombin auf Fibrinogen unter Bildung von Fibrinmonomeren, die ihrerseits unter Mitwirkung des aktivierten Faktors XIII das Fibrinnetz ausbilden.

Vorzugsweise kann gegebenenfalls zusätzlich zum Calciumcarbonat auch Magnesiumcarbonat und/oder Magnesiumoxid im insbesondere pastenförmigen Knochenersatzmaterial enthalten sein. Calciumcarbonat enthält in Abhängigkeit von der Herkunft fast immer Magnesiumcarbonat. Magnesium-lonen sind ebenso wie Calcium-lonen ein natürlicher Bestandteil des humanen Organismus.

Es ist auch zweckmäßig, dass gegebenenfalls ein oder mehrere Antibiotika oder andere pharmazeutische Wirkstoffe im Knochenersatzmaterial enthalten sind. Dieses Wirkstoffe können in gelöster als auch in nichtgelöster, suspendierter Form enthalten sein. Antibiotika aus der Gruppe der Aminoglykosid-Antibiotika sind auf Grund ihrer chemischen Stabilität bevorzugt. Besonders geeignet sind dabei die Breitbandantibiotika Gentamicinsulfat und Tobramycinsulfat. Daneben ist es auch möglich, Glykopeptid-Antibiotika, wie Vancomycin, Teicoplanin und Dalbavancin, im erfindungsgemäßen Knochenersatzmaterial einzubringen. Weiterhin können auch Fluorchinolon-Antibitika, wie Ofloxacin, Levofloxacin und Moxifloxacin, enthalten sein. Wenn Antibiotika in dem erfindungsgemäßen Knochenersatzmaterial enthalten sind, kann das Knochenersatzmaterial in vorteilhafter Weise auch bei der temporären Auffüllung von Knochenkavitäten genutzt werden, die bei der chirurgischen Sanierung von osteomyeltischen Knochenarealen entstehen.

Im Rahmen der Erfindung ist auch die Verwendung von anderen pharmazeutischen Wirkstoffen im Knochenersatzmaterial, wie Antiphlogistika, Kortikosteroide, und Knochenwachstumsfaktoren. Unter den Knochenwachstumsfaktoren sind rhBMP-2 und rhBMP-7 besonders bevorzugt.

Die Erfindung wird durch nachstehende Beispiele erläutert, ohne jedoch die Erfindung einzuschränken.

### Beispiel 1

Es wurde unter Verwendung eines Osmometers in 500 ml sterilem, pyrogenfreien Wasser bei 37 °C solange Calcium-L-lactat-Hydrat (Fluka) gelöst, bis die Lösung eine Osmolalität von 288 mOsmol hat. 100 ml dieser Lösung wurden anschließend mit 65,0 g Calciumcarbonat (Fluka) vermischt. Es entstand eine farblose bis hellgraue zähe Paste. 50 g dieser Paste wurden in eine konventionelle Kunststoff-Spritze eingebracht. Aus dieser konnte die Paste problemlos durch Betätigung des Kolbens herausgedrückt werden.

### Beispiel 2

Es wurden unter Verwendung eines Osmometers in 500 ml sterilem, pyrogenfreien Wasser bei 37°C solange Calciumacetat-Hydrat (Fluka) gelöst, bis die Lösung eine Osmolalit von 288 mOsmol hatte. 100 ml dieser Lösung wurden anschließend mit 60,0 g Calciumcarbonat (Fluka) vermischt. Es entstand eine farblose bis hellgraue zähe Paste. 50 g dieser Paste wurden in eine konventionelle Kunststoff-Spritze eingebracht. Aus dieser konnte die Paste problemlos durch Betätigung des Kolbens herausgedrückt werden.

## Patentansprüche

1. Knochenersatzmaterial, **dadurch gekennzeichnet, dass** es Calciumcarbonat enthält, welches als partikuläres Calciumcarbonat in einer wässrigen Lösung, die mindestens ein in Wasser lösliches Hämostyptikum enthält, suspendiert ist, wobei die wässrige Lösung eine solche Menge Hämostyptikum enthält, dass sie isotonisch ist,
und wobei das Hämostyptikum der Gruppe bestehend aus Calciumchlorid, Calciumacetat und Calciumlactat angehört.

2. Knochenersatzmaterial nach Anspruch 1, **dadurch gekennzeichnet, dass** es 55 bis 67 Masseprozent Calciumcarbonat enthält.

3. Knochenersatzmaterial nach Anspruch 1 oder 2, **dadurch gekennzeichnet, dass** das Calciumcarbonat eine Partikelgröße von 1-50 µm hat.

4. Knochenersatzmaterial nach einem der Ansprüche 1 bis 3, **dadurch gekennzeichnet, dass** es zusätzlich zum Calciumcarbonat auch Magnesiumcarbonat und/oder Magnesiumoxid enthält.

5. Knochenersatzmaterial nach einem der Ansprüche 1 bis 4, **dadurch gekennzeichnet, dass** es ein oder mehrere Antibiotika und/oder andere pharmazeutische Wirkstoffe enthält.

6. Knochenersatzmaterial nach einem der Ansprüche 1 bis 5, **dadurch gekennzeichnet, dass** es pastös ist.

## Claims

1. Bone replacement material, **characterized in that** it contains calcium carbonate suspended in the form of particulate calcium carbonate in an aqueous solution that contains at least one water-soluble hemostatic agent, whereby the aqueous solution contains an amount of the hemostatic agent to make it isotonic, and whereby the hemostatic agent belongs to the group consisting of calcium chloride, calcium acetate, and calcium lactate.

2. Bone replacement material according to claim 1, **characterized in that** it contains 55 to 67 % by mass calcium carbonate.

3. Bone replacement material according to claim 1 or 2, **characterized in that** the calcium carbonate is of particle size of 1-50 µm.

4. Bone replacement material according to any one of the claims 1 to 3, **characterized in that** it also contains magnesium carbonate and/or magnesium oxide in addition to calcium carbonate.

5. Bone replacement material according to any one of the claims 1 to 4, **characterized in that** it contains one or more antibiotics and/or other pharmaceutical agents.

6. Bone replacement material according to any one of the claims 1 to 5, **characterized in that** it is paste-like.

## Revendications

1. Matériau de substitut osseux **caractérisé en ce qu'**il contient du carbonate de calcium en suspension sous forme de carbonate de calcium particulaire dans une solution aqueuse qui contient au moins un hémostyptique hydrosoluble, la solution aqueuse contenant une quantité d'hémostyptique telle qu'elle est isotonique et l'hémostyptique appartenant au groupe consistant en le chlorure de calcium, l'acétate de calcium et le lactate de calcium.

2. Matériau de substitut osseux selon la revendication 1, **caractérisé en ce qu'**il contient 55 à 67 pourcent en masse de carbonate de calcium.

3. Matériau de substitut osseux selon la revendication 1 ou 2, **caractérisé en ce que** le carbonate de calcium a une taille de particules de 1-50 µm.

4. Matériau de substitut osseux selon l'une des revendications 1 à 3, **caractérisé en ce qu'**il contient en outre, en plus du carbonate de calcium, du carbonate de magnésium et/ou de l'oxyde de magnésium.

5. Matériau de substitut osseux selon l'une des revendications 1 à 4, **caractérisé en ce qu'**il contient un ou plusieurs antibiotiques et/ou d'autres substances actives pharmaceutiques.

6. Matériau de substitut osseux selon l'une des revendications 1 à 5, **caractérisé en ce qu'**il est pâteux.
